(19) Europäisches Patentamt
European Patent Office
Office européen des brevets

(11) EP 2 205 212 B1

(12) **EUROPÄISCHE PATENTSCHRIFT**

(45) Veröffentlichungstag und Bekanntmachung des Hinweises auf die Patenterteilung:
**23.09.2015 Patentblatt 2015/39**

(21) Anmeldenummer: **08848475.3**

(22) Anmeldetag: **06.11.2008**

(51) Int Cl.:
***A61K 8/22*** *(2006.01)* ***A61K 8/92*** *(2006.01)*
***A61Q 5/08*** *(2006.01)* ***A61Q 5/10*** *(2006.01)*
***A61K 8/37*** *(2006.01)* ***A61Q 5/04*** *(2006.01)*

(86) Internationale Anmeldenummer:
**PCT/EP2008/065041**

(87) Internationale Veröffentlichungsnummer:
**WO 2009/060020 (14.05.2009 Gazette 2009/20)**

(54) **MITTEL ENTHALTEND ESTERVERBINDUNG MIT WACHSALKOHOLKOMPONENTE**

AGENT CONTAINING ESTER COMPOUND HAVING A FATTY ALCOHOL COMPONENT

AGENTS CONTENANT UN COMPOSÉ ESTER QUI COMPORTE UN COMPOSANT ALCOOL DE CIRE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **09.11.2007 DE 102007053952**

(43) Veröffentlichungstag der Anmeldung:
**14.07.2010 Patentblatt 2010/28**

(73) Patentinhaber: **Henkel AG & Co. KGaA**
**40589 Düsseldorf (DE)**

(72) Erfinder:
- **KLEEN, Astrid**
  **20457 Hamburg (DE)**
- **RATH, Susanne**
  **20359 Hamburg (DE)**
- **LISSNER, Yvonne**
  **21073 Hamburg (DE)**

(56) Entgegenhaltungen:
**DE-C1- 19 723 538** **US-A- 4 844 886**
**US-A1- 2004 181 883** **US-A1- 2005 039 270**
**US-A1- 2006 121 070** **US-A1- 2007 033 744**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 205 212 B1

## Beschreibung

Die vorliegende Anmeldung betrifft wässrige Wasserstoffperoxidzubereitungen, die mindestens eine spezielle Esterverbindung enthalten sowie deren Verwendung in der Färbung keratinischer Fasern. Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe sowie der Veränderung der Form des Kopfhaares eine herausragende Rolle.

Die Veränderung der Form der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Dabei können Dauerwell- und andere die Haarform verändernde Verfahren nahezu unabhängig vom Typ der zu behandelnden Haare eingesetzt werden. Gleichzeitig oder alternativ zu der Formveränderung besteht aber auch vielfach der Wunsch, die Haarfarbe zu ändern und zusammen mit der Haarfärbung auch eine Aufhellung des zu färbenden Haares zu erreichen.

Konventionelle Haarfärbemittel enthalten als farbverändernde Wirkstoffe in der Regel mindestens eine Entwickler- und/oder mindestens eine Kupplerkomponenten und gegebenenfalls noch direktziehende Farbstoffe als Nuanceure. Kuppler- und Entwicklerkomponenten werden auch als Oxidationsfarbstoffvorprodukte bezeichnet. Die Grundlagen der Blondierverfahren sind dem Fachmann bekannt. Vor ihrer Anwendung auf menschliches Haar werden üblicherweise Haarfärbe- und/oder - aufhellungsmittel in fester oder pastöser Form mit einer verdünnten wässrigen WasserstoffperoxidLösung vermischt. Die resultierende Mischung wird dann auf das Haar aufgebracht und nach einer bestimmten Einwirkzeit wieder ausgespült. Die Einwirkungsdauer auf dem Haar zur Erzielung einer vollständigen Ausfärbung beziehungsweise Aufhellung liegt in der Regel zwischen etwa 30 und 40 Minuten. Es ist nahe liegend, dass bei den Benutzern dieser Haarfarben oder Blondiermittel ein Bedürfnis besteht, diese Einwirkungszeit zu verringern.

Um eine ausreichenden Färbe- und/oder Blondierwirkung zu erzielen, sind derartige Mittel üblicherweise stark alkalisch eingestellt, der pH-Wert liegt dabei zwischen 8 und 10,5. Derart hohe pH-Werte sind erforderlich, um eine Öffnung der äußeren Schuppenschicht (Cuticula) zu gewährleisten und somit eine Penetration der aktiven Spezies (Wasserstoffperoxid) ins Haar zu ermöglichen.

Weder die pastenförmigen noch die pulverförmigen Färbe- und/oder Blondiermittel, die heute auf dem Markt sind, können als optimal angesehen werden. So besteht weiterhin das Bedürfnis intensiverere Färbungen zu erzielen beziehungsweise mit geringeren Wirkstoffmengen gleich bleibende Ergebnisse zu erzielen.

Aufgrund der stark alkalischen Bedingungen sowie des Einsatzes von Wasserstoffperoxid kann es im Zusammenhang mit dem farbverändernden Vorgang zu Reizungen der Kopfhaut kommen. Diese sind sowohl während der Einwirkung des farbverändernden Mittels als auch im Anschluss an die Behandlung zu beobachten.

Daher hat es in der Vergangenheit nicht an Versuchen gefehlt, die farbverändernden Mittel einerseits hinsichtlich ihrer Kopfhaut- und Haarverträglichkeit zu optimieren und andererseits Wirkstoff zu entwickeln, die gleichartige Ergebnisse bei geringerer Konzentration der reizenden Stoffe ermöglichen. Dennoch konnten die Mittel des Standes der Technik die bestehenden Anforderungen bisher nicht vollständig erfüllen.

Ein großes Problem bei der Entwicklung geeigneter Wirkstoffe stellen die hohen Anforderungen hinsichtlich ihrer Stabilität dar, da einerseits die Färbecremes üblicherweise einen hohen pH-Wert und andererseits die Oxidationsmittelzubereitungen einen niedrigen pH-Wert aufweisen. Auch müssen diese Wirkstoffe gegenüber den oxidativen Bedingungen im Zusammenhang mit den Färbungen stabil sein. Ferner sind Unverträglichkeiten der verschiedenen Wirkstoffe untereinander und somit eine geringe Lagerstabilität zu vermeiden.

Es bestand daher weiterhin Bedarf an Wirkstoffen, die unter den jeweiligen Bedingungen die Wirkungen der jeweiligen Mittel positiv verstärken können. Im Fall der oxidativen Haarfärbung könnten auf diese Weise entweder intensivere Färbungen oder eine Verringerung der Konzentration von beispielsweise Farbstoffvorprodukten und/oder Wasserstoffperoxid ermöglicht werden.

DE 19723538C1 offenbart Mittel zum Entfärben oder Blondieren von Haaren, die unmittelbar vor der Anwendung durch Vermischen einer cremeförmigen Blondiermittelsuspension mit einer wässrigen Wasserstoffperoxidlösung oder Wasserstoffperoxidemulsion hergestellt werden, wobei die Blondiermittelsuspension einen flüssigen oder wachsförmigen langkettigen hydrophoben Fettsäureester oder synthetischen Bienenwachsersatzstoff enthält.

US 2004/0181883 A1 und US 2007/0033744 A1 offenbaren Haarfärbemittel, die Bienenwachs und wässriges Wasserstoffperoxid enthalten.

US 4844886 offenbart Mittel zur oxidativen Haarfärbung oder zur Dauerwellfixierung, die Wollwachs und wässriges Wasserstoffperoxid enthalten.

US 2006/0121070 A1 offenbart ein kationisches cremförmiges Oxidationshaarfärbemittel, das Bienenwachs enthält und mit einer wässrigen Wasserstoffperoxidlösung vermischt wird.

US 2005/0039270 A1 offenbart ein Haarbleichmittel, das Bienenwachs enthält und mit einer wässrigen Wasserstoffperoxidlösung vermischt wird.

Es wurde nunmehr überraschenderweise gefunden, dass wässrige Wasserstoffperoxidzubereitungen, die mindestens eine spezielle Esterverbindung enthalten, stabil sind, hinsichtlich ihrer Kopfhautverträglichkeit zufrieden stellend sind und gleichzeitig eine Verbesserung der Farbveränderung ermöglichen. Darüber hinaus weisen die mit den erfindungsgemäßen Zubereitungen behandelten Fasern einen besseren Pflegezustand auf.

Ein erster Gegenstand der vorliegenden Anmeldung ist daher ein Kit zur Färbung keratinischer Fasern, bestehend aus einer ersten Zubereitung (A), enthaltend mindestens eine Entwicklerkomponente und mindestens eine Kupplerkomponente, und einer zweiten Zubereitung (B) in Form einer wässrigen Wasserstoffperoxidzubereitung, umfassend mindestens eine Esterverbindung, die als Alkoholkomponente einen gesättigten oder ungesättigten, verzweigten oder unverzweigten Wachsalkohol enthält.

Die Zubereitungen (B) des erfindungsgemäßen Kits enthalten als wesentliche Komponente mindestens eine Esterverbindung, die als Alkoholkomponente einen gesättigten oder ungesättigten, verzweigten oder unverzweigten Wachsalkohol enthält.

Dabei haben sich insbesondere die gesättigten Wachsalkohole als bevorzugt erwiesen. Weiterhin haben sich die unverzweigten Wachsalkohole als bevorzugt erwiesen. Besonders bevorzugt sind die gesättigten und unverzweigten Wachsalkohole. Ebenfalls bevorzugt sind Wachsalkohole mit einer endständigen Hydroxygruppe.

Beispiele für erfindungsgemäß bevorzugte Wachsalkohole sind Tetracosan-1-ol, Hexacosan-1-ol, Triacontan-1-ol sowie Hentriacontan-1-ol. Triacontan-1-ol ist erfindungsgemäß ganz besonders bevorzugt.

Als weiteren Bestandteil enthält die erfindungsgemäße Esterverbindung mindestens eine Fettsäurekomponente.

Die Fettsäurekomponente weist vorzugsweise 10 bis 30 Kohlenstoffatome, insbesondere 16 bis 26 Kohlenstoffatome auf. Dabei sind sowohl gesättigte als auch ungesättigte sowie verzweigte und unverzweigte Fettsäuren prinzipiell geeignet. Als bevorzugte Fettsäuren kommen insbesondere die unverzweigten gesättigten Fettsäuren, wie beispielsweise Tetradecansäure, Pentadecansäure, Palmitinsäure, Margarinsäure, Stearinsäure, Nonadecansäure, Arachinsäure, Behensäure, Tetracosansäure, Cerotinsäure sowie Triacontansäure in Frage. Palmitinsäure, Stearinsäure, Behensäure und Triacontansäure sind erfindungsgemäß besonders bevorzugt.

Besonders vorteilhaft im Sinne der vorliegenden Erfindung ist es ferner, wenn mindestens zwei verschiedene Esterverbindungen in den Zubereitungen (B) des erfindungsgemäßen Kits enthalten sind, wobei die erste Esterverbindung eine Fettsäurekomponente mit 12 bis 18 Kohlenstoffatomen und die zweite Esterverbindung eine Fettsäurekomponente mit 22 bis 30 Kohlenstoffatomen aufweist.

Ein Beispiel für eine derartige Kombination sind Esterverbindungen auf Basis von Palmitinsäure und Cerotinsäure. Ganz besonders bevorzugte Esterverbindungen sind die Ester von Triacontan-1-ol mit Palmitinsäure und Cerotinsäure. Derartige Esterverbindungen oder auch deren Mischungen können beispielsweise aus natürlichem Bienenwachs gewonnen werden.

Es hat sich aber erfindungsgemäß auch als vorteilhaft erwiesen, wenn das komplexe Gemisch des natürlichen Bienenwachses, das eine erfindungsgemäße Esterverbindung umfasst, als solches in den Zubereitungen (B) des erfindungsgemäßen Kits zum Einsatz kommt.

Neben der erfindungswesentlichen Esterverbindung enthalten die Zubereitungen (B) des erfindungsgemäßen Kits zwingend Wasserstoffperoxid und dessen Zerfallsprodukte. Als Quelle für Wasserstoffperoxid können ebenfalls dessen Anlagerungsprodukte beispielsweise an Harnstoff, Melamin oder Natriumborat verwendet werden. Erfindungsgemäß ist es ebenfalls denkbar, dass das Wasserstoffperoxid und dessen Zerfallsprodukte aus anorganischen oder organischen Peroxidsalzen, beispielsweise durch den Einsatz von Natriumperoxid, gebildet werden.

Die Zubereitungen (B) des erfindungsgemäßen Kits enthalten Wasserstoffperoxid vorzugsweise in einer Menge von 0,1 bis 20 Gew.-%, vorzugsweise von 2 bis 12 Gew.%.

Als Aktivatoren für Wasserstoffperoxid und dessen Zerfallsprodukte können die Zubereitungen (B) des erfindungsgemäßen Kits in einer bevorzugten Ausführungsform weiterhin Carbonatsalze, bzw. Hydrogencarbonatsalze enthalten. Diese sind bevorzugt ausgewählt aus der Gruppe der Ammonium-, Alkali- (insbesondere Natrium- und Kalium-), sowie Erdalkali- (insbesondere Calcium-) -carbonatsalze bzw. -hydrogencarbonatsalze. Besonders bevorzugte Carbonat- bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Aktivatoren verwendet werden.

Die Zubereitungen (B) des erfindungsgemäßen Kits werden zur oxidativen Entwicklung von Haarfärbungen verwendet. Dabei hat es sich gezeigt, dass diese Zubereitungen (B) auch zur Steigerung der Farbintensität oxidativer Färbungen verwendet werden können.

Unter "keratinischen Fasern" sind erfindungsgemäß Pelze, Wolle, Federn und insbesondere menschliche Haare zu verstehen.

Hinsichtlich der in den Zubereitungen (A) des erfindungsgemäßen Kits einsetzbaren Farbstoffvorprodukte unterliegt die vorliegende Erfindung keinerlei Einschränkungen. Die erfindungsgemäßen Färbemittel enthalten als Farbstoffvorprodukte

- Oxidationsfarbstoffvorprodukte vom Entwickler- und vom Kuppler-Typ,
- und optional Vorstufen naturanaloger Farbstoffe, wie Indol- und Indolin-Derivate,

sowie Mischungen von Vertretern dieser Gruppen.

Im Rahmen einer ersten Ausführungsform der vorliegenden Erfindung enthalten die erfindungsgemäßen Mittel mindestens ein Farbstoffvorprodukt vom Entwickler- und eins vom Kupplertyp.

Es kann erfindungsgemäß bevorzugt sein, als Entwicklerkomponente ein p-Phenylendiaminderivat oder eines seiner physiologisch verträglichen Salze einzusetzen.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Besonders bevorzugte Pyrimidin-Derivate sind insbesondere die Verbindungen 2,4,5,6-Tetraamino-pyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Im Folgenden werden Beispiele für die als Substituenten der Verbindungen der Formeln (E1) bis (E6) genannten Reste aufgezählt:

Kupplerkomponenten bilden im Rahmen der oxidativen Färbung allein keine signifikante Färbung aus, sondern benötigen stets die Gegenwart von Entwicklerkomponenten. Daher ist es erfindungsgemäß, dass bei Verwendung mindestens einer Entwicklerkomponente zusätzlich mindestens eine Kupplerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:

- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und den physiologisch verträglichen Salzen aller vorstehend genannten Verbindungen.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Besonders bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie 5,6-Dihydroxyindolin-2-carbonsäure.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Weiterhin können die Mittel zusätzlich zu den Farbstoffvorprodukten mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

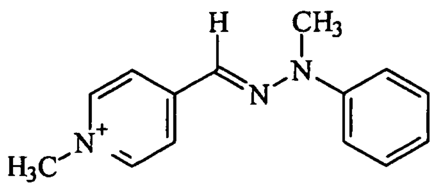

(DZ1)

$CH_3SO_4^-$

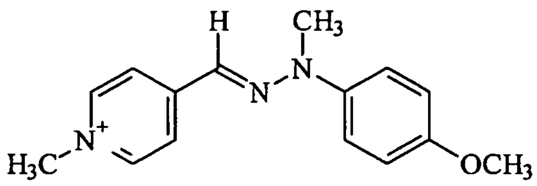

(DZ2)

$Cl^-$

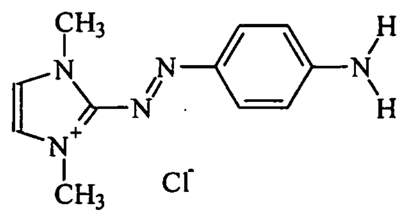

(DZ3)

$Cl^-$

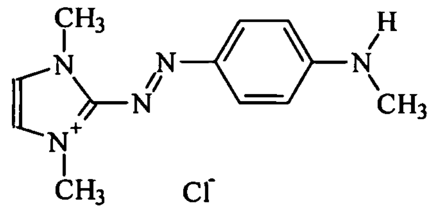

(DZ4)

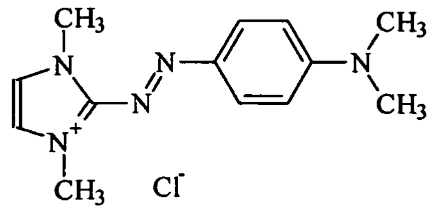

(DZ5)

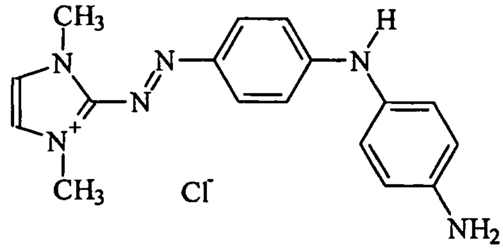

(DZ6)

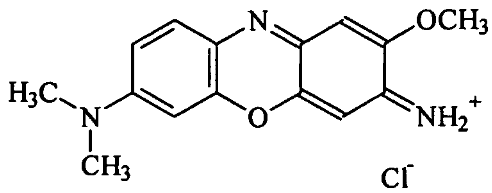

(DZ7)

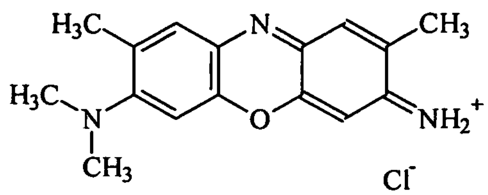

(DZ8)

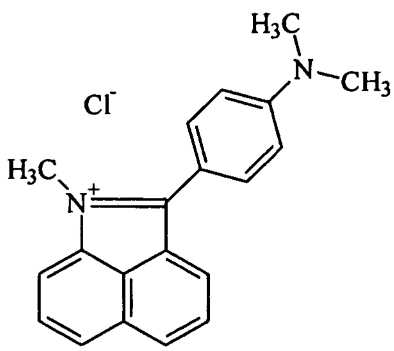

(DZ9)

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe. Nichtionische direktziehende Farbstoffe:

Als nichtionische direktziehende Farbstoffe eignen sich insbesondere nichtionische Nitro- und Chinonfarbstoffe und neutrale Azofarbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-{(4-amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in unter-geordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel enthalten die direktziehenden Farbstoffe bevorzugt in einer Menge von 0,01 bis 20 Gew.-%, bezogen auf die gesamte Anwendungszubereitung.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Im Rahmen einer bevorzugten Ausführungsform der vorliegenden Erfindung enthält die Zubereitung (A) weiterhin mindestens ein Flavonoid der Formel (I)

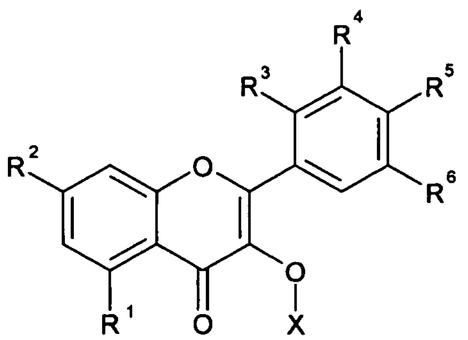

**(I)**

wobei $R^1$ bis $R^6$ stehen unabhängig voneinander für ein Wasserstoffatom, eine Hydroxygruppe, ein Hydroxy-($C_1$-bis $C_4$)-alkoxygruppe oder eine Sulfatgruppe und X steht für einen gegebenenfalls substituierten Glucosidrest, enthält.

Erfindungsgemäß bevorzugte Hydroxy-($C_1$-bis $C_4$)-alkoxygruppen sind die Hydroxymethoxygruppe, die 2-Hydroxyethoxygruppe, die 3-Hydroxypropoxygruppe, die 2-Hydroxypropoxygruppe, die 4-Hydroxybutoxygruppe, die 3-Hydroxybutoxygruppe sowie die 2-Hydroxybutoxygruppe. Die 2-Hydroxyethoxygruppe ist eine ganz besonders bevorzugte Hydroxy-($C_1$-bis $C_4$)-alkoxygruppe. Erfindungsgemäß haben sich Flavonoide der Formel (I) als bevorzugt erwiesen, bei denen mindestens zwei der Reste $R^1$ bis $R^6$ für ein Wasserstoffatom stehen, besonders bevorzugt sind Flavonoide der Formel (I), bei denen zwei oder drei der Reste $R^1$ bis $R^6$ für ein Wasserstoffatom stehen.

Weiterhin sind Flavonoide der Formel (I) bevorzugt, bei denen $R^1$ für eine Hydroxygruppe steht. Ebenfalls bevorzugt sind Flavonoide, bei denen die Reste $R^1$ bis $R^6$ maximal für 3 voneinander verschiedene Substituenten stehen, vorzugsweise lediglich für 2 voneinander verschiedene Substituenten.

Besonders bevorzugt sind Flavonoide der Formel (I), bei denen $R^1$, $R^2$ und $R^5$ für eine Hydroxygruppe und $R^3$, $R^4$ und $R^6$ für ein Wasserstoffatom stehen.

Weiterhin besonders bevorzugt sind Flavonoide der Formel (I), bei denen $R^1$ für eine Hydroxygruppe steht, $R^2$, $R^4$ und $R^5$ für eine Sulfatgruppe stehen und $R^3$ und $R^6$ für ein Wasserstoffatom stehen. Ebenso besonders bevorzugt sind Flavonoide der Formel (I), bei denen $R^1$ für eine Hydroxygruppe steht, $R^2$, $R^4$ und $R^5$ für eine 2-Hydroxyethoxygruppe stehen und $R^3$ und $R^6$ für ein Wasserstoffatom stehen. Ganz besonders bevorzugt sind Flavonoide der Formel (I), die ein durch die Gruppe X substituiertes Derivat von Flavonol, Galangin, Fisetin, Kaempferol, Quercetin, Morin, Robinetin, Gossypetin oder Myricetin sind.

Im Rahmen der erfindungsgemäßen Flavonoide der Formel (I) steht der Rest X für einen Glucosidrest. Dies ist erfindungsgemäß nicht auf Derivate der Glucose beschränkt, sondern steht ganz allgemein für $\alpha$-glucosidisch oder $\beta$-glucosidisch gebildete Acetale auch anderer Zuckerkomponenten. Erfindungsgemäß bevorzugt haben sich Flavonoide der Formel (I) erwiesen, bei denen X für einen Rest der Formel (II)

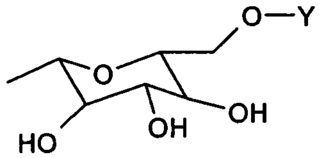

(II)

steht, wobei die Gruppe -O-Y für einen über eine Etherfunktion verknüpften weiteren Monosaccharidrest oder für einen über eine Esterfunktion verknüpften organischen aromatischen Säurerest steht. Als erfindungsgemäße Monosaccharide kommen vorzugsweise sowohl Pentosen als auch Hexosen in Frage. Erfindungsgemäß bevorzugte Monosaccharidreste sind Ribose, Xylose, Arabinose, Glucose, Mannose, Galactose, Fructose, Sorbose, Fucose und Rhamnose. In einigen Fällen hat es sich als besonders bevorzugt erwiesen, wenn der Rest -O-Y für einen über eine Etherfunktion verknüpften 6-Desoxyzuckerrest, insbesondere einen Fucoserest oder einen Rhamnoserest, ganz besonders für einen Rhamnoserest, steht

Im Rahmen einer anderen Ausführungsform der vorliegenden Erfindung steht die Gruppe -O-Y einen über eine Esterfunktion verknüpften organischen aromatischen Säurerest. Erfindungsgemäß bevorzugte gegebenenfalls substituierten Zimtsäurerest sind beispielsweise der o-Cumarsäurerest, der p-Cumarsäurerest und deren substituierten Derivate; der p-Cumarsäurerest ist ein ganz besonders bevorzugter Rest einer erfindungsgemäßen organischen aromatischen Säure. Erfindungsgemäß ganz bsonders bevorzugte Flavonoide der Formel (I) sind

- Tilirosid ([(2R,3R,4S,5R,6S)-6-[5,7-dihydroxy-2-(4-hydroxyphenyl)-4-oxo-chromen-3 -yl]oxy-3,4,5-trihydroxy-oxan-2-yl]methyl (E)-3-(4-hydroxyphenyl)prop-2-enoate; INCI-Bezeichnung: Tiliroside, CAS-Nr. 20316-62-5).
- Rutinsulfat (3,3',4', 4,7-Pentahydroxyflavon-3-rhamnoglucosid, INCI-Bezeichnung Disodium Rutinyl Disulfate),
- Troxerutin (2-[3,4-bis(2-hydroxyethoxy)phenyl]-5-hydroxy-7-(2-hydroxyethoxy)-3-[3, 4,5-trihydroxy-6-[(3,4,5-trihydroxy-6-methyl-oxan-2-yl)oxymethyl]oxan-2-yl]oxy-chromen-4-one; INCI-Bezeichnung: Troxerutin, CAS-Nr. 96304-71-1) und
- Isoquercetin (2-(3,4-dihydroxyphenyl)-5,7-dihydroxy-3[(2S,3R,4S,5R,6R)-3,4,5-trihydroxy-6-(hydroxymethyl)oxan-2-yl]oxy-chromen-4-one; CAS-Nr. 482-35-9).

Ein erfindungsgemäß ganz besonders bevorzugtes Flavonoid ist Tiliroside.

In der WO 02/69926 wird angegeben, dass Tilirosid beispielsweise aus den Pflanzen der Gattung Althaea, Aristolochia, Helianthemum, Lindera, Magnolia, Platanus, Potentilla, Quercus, Rosa, Sida, Sorbus und/oder Tilia gewonnen werden kann, wobei folgende Arten bevorzugt sind: Althaea officinalis, Althaea rosea, Aristolochia heterophylla, Helianthemum glomeratum, Lindera megaphyfla, Magnolia salicifolia, Platanus acerifolia, Platanus occidentalis, Potentilla anserina, Quercus pubescens, Quercus suber, Quercus laurifolia, Quercus ilex, Quercus imbricaria, Quercus virginiana, Rosa pomifera, Sida rhombifolia, Sida poeppigiana, Sida cordifolia, Sida glaziovii, Sorbus pendula, Tilia argenta und Tilia cordata.

In der WO-06/099930 wird weiterhin beschrieben, dass Tilirosid aus Pflanzen der Familie der Sterculiaceae gewonnen werden kann. Als bevorzugt werden dort die Waitheria Spezies, vorzugsweise Waitheria americana, Waitheria douradinha, Waitheria panicucata, Waitheria indica, Waitheria viscosissima, Waitheria antennalis, Waitheria ovata, Waitheria tornentosa, Waitheria madagascariensis, Waitheria glornerata, Waitheria bicolor, Waitheria fryxellii, Waitheria tundeltiana, Waitheria tridentata, Waitheria operculata, Waitheria bracteosa, Waitheria douradinha, Waitheria macropoda, Waitheria caroliniana, Waltheria arenicola, Waitheria melochia, Waitheria acurninata, Waitheria theobroma, Waitheria indivia oder Waitheria taiwana genannt.

Entsprechende Verfahren zur Gewinnung des Rohstoffs sind in den beiden genannten Schriften offenbart.

Tilirosid wird als kosmetischer Wirkstoff beispielsweise unter der Handelsbezeichnung Ronacare® Tiliroside von der Fa. Merck kommerziell vertrieben. Es handelt sich dabei um eine Mischung von ca. 5Gew.-% reines Tilirosid

mit ca. 95Gew.% Sorbitol.

Auch wenn die Abmischung mit Sorbitol erfindungsgemäß bevorzugt ist, ist auch ein Einsatz von reinem Tilirosid oder in Kombination mit anderen pulverförmigen Stellmitteln erfindungsgemäß möglich.

Die Flavonoide der Formel (I) sind in den erfindungsgemäßen Zubereitungen vorzugsweise in einer Menge von 0,0001 bis 5Gew.-%, insbesondere von 0,002 bis 1Gew.-%, jeweils bezogen auf den reinen Wirkstoff und das gesamte anwendungsbereite Mittel, enthalten.

Im Rahmen einer weiteren bevorzugten Ausführungsform enthält die erfindungsgemäße Zubereitung als Pflegestoff mindestens ein kationisches Tensid.

Die kationischen Tenside sind in den erfindungsgemäßen Zubereitungen bevorzugt in Mengen von 0,05 bis 10 Gew.%, bezogen auf die gesamte Anwendungszubereitung, enthalten. Mengen von 0,1 bis 5 Gew.% sind besonders bevorzugt.

Im Rahmen einer weiteren bevorzugten Ausführungsform der vorliegenden Erfindung enthält die Zubereitung (A) als Pflegestoff mindestens ein pflegendes Polymer.

Weiterhin sind kationiserte Proteinhydrolysate zu den kationischen Polymeren zu zählen

Die erfindungsgemäßen Zubereitungen enthalten die kationischen Polymere bevorzugter Weise in einer Menge von 0,01 bis 5 Gew.%, insbesondere in einer Menge von 0,1 bis 2 Gew.%, jeweils bezogen auf die gesamte Anwendungszubereitung.

Im Rahmen einer weiteren bevorzugten Ausführungsform enthalten die erfindungsgemäßen Zubereitungen mindestens einen UV-Filter. Die erfindungsgemäß geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Bevorzugt sind solche UV-Filter, deren molarer Extinktionskoeffizient am Absorptionsmaximum oberhalb von 15 000, insbesondere oberhalb von 20000, liegt.

Weiterhin wurde gefunden, dass bei strukturell ähnlichen UV-Filtern in vielen Fällen die wasserunlösliche Verbindung im Rahmen der erfindungsgemäßen Lehre die höhere Wirkung gegenüber solchen wasserlöslichen Verbindungen aufweist, die sich von ihr durch eine oder mehrere zusätzlich ionische Gruppen unterscheiden. Als wasserunlöslich sind im Rahmen der Erfindung solche UV-Filter zu verstehen, die sich bei 20 °C zu nicht mehr als 1 Gew.-%, insbesondere zu nicht mehr als 0,1 Gew.-%, in Wasser lösen. Weiterhin sollten diese Verbindungen in üblichen kosmetischen Ölkomponenten bei Raumtemperatur zu mindestens 0,1, insbesondere zu mindestens 1 Gew.-% löslich sein). Die Verwendung wasserunlöslicher UV-Filter kann daher erfindungsgemäß bevorzugt sein.

Zwei bevorzugte UV-Filter mit kationischen Gruppen sind die als Handelsprodukte erhältlichen Verbindungen Zimtsäureamidopropyl-trimethylammoniumchlorid (Incroquat®UV-283) und Dodecyldimethylaminobenzamidopropyl-dimethylammoniumtosylat (Escalol® HP 610).

Selbstverständlich umfasst die erfindungsgemäße Lehre auch die Verwendung einer Kombination von mehreren UV-Filtern. Im Rahmen dieser Ausführungsform ist die Kombination mindestens eines wasserunlöslichen UV-Filters mit mindestens einem UV-Filter mit einer kationischen Gruppe bevorzugt. Die UV-Filter sind in den erfindungsgemäßen Mitteln üblicherweise in Mengen 0,01-5 Gew.-%, bezogen auf die gesamte Anwendungszubereitung, enthalten. Mengen von 0,1-2,5 Gew.-% sind bevorzugt.

Im Rahmen einer weiteren bevorzugten Ausführungsform enthalten die erfindungsgemäßen Zubereitungen als Pflegestoff mindestens ein Vitamin, ein Provitamin, eine Vitaminvorstufe sowie eines derer Derivate.

Dabei sind erfindungsgemäß solche Vitamine, Pro-Vitamine und Vitaminvorstufen bevorzugt, die üblicherweise den Gruppen A, B, C, E, F und H zugeordnet werden.

Vitamin H. Als Vitamin H wird die Verbindung (3aS,4S, 6a*R*)-2-Oxohexahydrothienol[3,4-*d*]-imidazol-4-valeriansäure bezeichnet, für die sich aber inzwischen der Trivialname Biotin durchgesetzt hat. Biotin ist ein erfindungsgemäß ganz besonders bevorzugtes Vitamin. Durch Einsatz von Biotin in den erfindungsgemäßen Zubereitungen konnte überraschender Weise die Restrukturierung der Fasern verbessert werden, eine Strukturstabilisierung erzielt werden sowie das Reizpotential der Mittel wesentlich reduziert werden. Biotin ist in den erfindungsgemäßen Zubereitungen bevorzugt in Mengen von 0,0001 bis 2,0 Gew.%, insbesondere in Mengen von 0,001 bis 0,2 Gew.%, jeweils bezogen auf die gesamte Anwendungszubereitung enthalten.

Bevorzugt enthalten die erfindungsgemäßen farbverändernden Mittel Vitamine, Provitamine und Vitaminvorstufen aus den Gruppen A, B, C, E und H. Das Vitamin C kann erfindungsgemäß ganz besonders bevorzugt sein.

Panthenol, Pantolacton, Pyridoxin und seine Derivate sowie Nicotinsäureamid und Biotin sind besonders bevorzugt.

Im Rahmen einer weiteren bevorzugten Ausführungsform enthalten die erfindungsgemäßen Zubereitungen mindestens einen Pflanzenextrakt.

Üblicherweise werden diese Extrakte durch Extraktion der gesamten Pflanze hergestellt. Es kann aber in

einzelnen Fällen auch bevorzugt sein, die Extrakte ausschließlich aus Blüten und/oder Blättern der Pflanze herzustellen.

Hinsichtlich der erfindungsgemäß bevorzugten Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Besonders bevorzugt sind die Extrakte aus Moringa Olifeira, Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Moringa Olifeira, Grünem Tee, Mandel, Aloe Vera, Kokosnuss, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Zubereitungen Mischungen aus mehreren, insbesondere aus zwei, verschiedenen Pflanzenextrakten einzusetzen.

Im Rahmen einer weiteren Ausführungsform enthalten die erfindungsgemäßen Zubereitungen als Pflegestoff mindestens eine Carbonsäure.

Als Beispiele für erfindungsgemäße Carbonsäuren seien genannt Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, eine Dicarbonsäure ausgewählt aus der Gruppe, die gebildet wird durch Verbindungen der allgemeinen Formel (N-I),

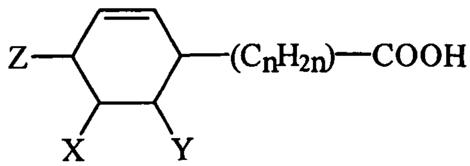

**(N-I)**

in der Z steht für eine lineare oder verzweigte Alkyl- oder Alkenylgruppe mit 4 bis 12 Kohlenstoffatomen, n für eine Zahl von 4 bis 12 sowie eine der beiden Gruppen X und Y für eine COOH-Gruppe und die andere für Wasserstoff oder einen Methyl- oder Ethylrest, Dicarbonsäuren der allgemeinen Formel (N-I), die zusätzlich noch 1 bis 3 Methyl- oder Ethylsubstituenten am Cyclohexenring tragen sowie Dicarbonsäuren, die aus den Dicarbonsäuren gemäß Formel (N-I) formal durch Anlagerung eines Moleküls Wasser an die Doppelbindung im Cyclohexenring entstehen.

Weiterhin ist es erfindungsgemäß bevorzugt 2-Pyrrolidinon-5-carbonsäure und deren Derivate als Carbonsäure einzusetzen. Besonders bevorzugt sind die Natrium-, Kalium-, Calcium-, Magnesium- oder Ammoniumsalze, bei denen das Ammoniumion neben Wasserstoff eine bis drei $C_1$- bis $C_4$-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf die gesamte Anwendungszubereitung, besonders bevorzugt 0,1 bis 5, und insbesondere 0,1 bis 3 Gew.%.

Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der ß-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von $C_{12}$-$C_{15}$-Fettalkoholen besonders bevorzugt. Ester dieses Typs sind im Handel erhältlich, z.B. unter dem Warenzeichen Cosmacol® der EniChem, Augusta Industriale. Besonders bevorzugte Polyhydroxypolycarbonsäuren sind Polymilchsäure und Polyweinsäure sowie deren Ester.

Im Rahmen einer weiteren bevorzugten Ausführungsform enthalten die erfindungsgemäßen Zubereitungen

als Pflegestoff mindestens ein Proteinhydrolysat und eines seiner Derivate.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex), Hydrosoy® (Croda), Hydrolupin® (Croda), Hydrosesame® (Croda), Hydrotritium® (Croda) und Crotein® (Croda) erhältlich. Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon® (Cognis), Lexein® (Inolex), Crolastin® (Croda), Crosilk®(Croda) oder Crotein® (Croda) vertrieben.

Selbstverständlich umfasst die erfindungsgemäße Lehre alle isomeren Formen, wie cis - trans-Isomere, Diastereomere und chirale Isomere.

Erfindungsgemäß ist es auch möglich, eine Mischung aus mehreren Proteinhydrolysaten einzusetzen. Die Proteinhydrolysate sind in den erfindungsgemäßen farbverändernden Mitteln in Konzentrationen von 0,01 Gew. -% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Im Rahmen einer weiteren bevorzugten Ausführungsform enthalten die erfindungsgemäßen Zubereitungen als Pflegestoff Ectoin oder Ectoinderivate, Allantoin, Taurin und Bisabolol. Erfindungsgemäß werden unter dem Begriff "Ectoin und Ectoinderivate" Verbindungen der Formel (IV)

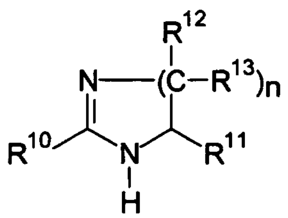 (IVa) 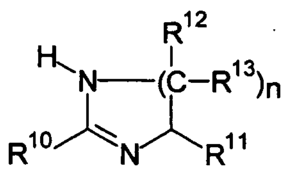 (IVb)

und/oder deren physiologisch verträglichen Salzes und/oder einer isomeren oder stereoisomeren Form verstanden, wobei

- $R^{10}$ steht für ein Wasserstoffatom, einen verzweigten oder unverzweigten $C_1$ - $C_4$-Alkylrest oder einen $C_2$ - $C_4$-Hydroxyalkylrest,
- R" steht für ein Wasserstoffatom, eine Gruppierung -$COOR^{14}$ oder eine Gruppierung-$CO(NH)R^{14}$, wobei $R^{14}$ für ein Wasserstoffatom, einen $C_1$ - $C_4$-Alkylrest, einen Aminosäurerest, einen Dipeptid- oder einen Tripeptidrest stehen kann,
- $R^{12}$ und $R^{13}$ stehen unabhängig voneinander für ein Wasserstoffatom, einen $C_1$ - $C_4$-Alkylrest oder einer der beiden Reste steht für eine Hydroxygruppe und
- n steht für eine ganze Zahl von 1 bis 3.

Geeignete physiologisch verträgliche Salze der allgemeinen Verbindungen gemäß der Formel (IVa) oder (IVb) sind beispielsweise die Alkali-, Erdalkali-, Ammonium-, Triethylamin- oder Tris-(2-hydroxyethyl)aminsalze sowie solche, die sich aus der Umsetzung von Verbindungen gemäß der Formel (IVa) oder (IVb) mit anorganischen und organischen

Säuren wie Salzsäure, Phosphorsäure, Schwefelsäure, verzweigten oder unverzweigten, substituierten oder unsubstituierten (beispielsweise durch eine oder mehrere Hydroxygruppen) $C_1$ - $C_4$- Mono- oder Dicarbonsäuren, aromatische Carbonsäuren und Sulfonsäuren wie Essigsäure, Citronensäure, Benzoesäure, Maleinsäure, Fumarsäure, Weinsäure und p-Toluolsulfonsäure ergeben. Beispiele für besonders bevorzugte physiologisch verträgliche Salze sind die Na-, K-, Mg- und Ca- und Ammoniumsalze der Verbindungen gemäß der Formel (IVa) oder (IVb), sowie die Salze, die sich durch Umsetzung von Verbindungen gemäß der Formel (IVa) oder (IVb) mit Salzsäure, Essigsäure, Citronensäure und Benzoesäure ergeben.

Unter isomeren oder stereoisomeren Formen der Verbindungen gemäß Formel (IVa) oder (IVb) werden erfindungsgemäß alle auftretenden optischen Isomere, Diastereomere, Racemate, Zwitterionen, Kationen oder Gemische davon verstanden.

Unter dem Begriff Aminosäure werden die stereoisomeren Formen, z.B. D- und L-Formen, folgender Verbindungen verstanden:

> Asparagin, Arginin, Asparaginsäure, Glutamin, Glutaminsäure, β-Alanin, γ-Aminobutyrat, $N_\varepsilon$-Acetyllysin, $N_\delta$-Acetylornitin, $N_\gamma$-Acetyldiaminobutyrat, $N_\alpha$-Acetyldiaminobutyrat, Histidin, Isoleucin, Leucin, Methionin, Phenylalanin, Serin, Threonin und Tyrosin.

L-Aminosäuren sind bevorzugt. Aminosäurereste leiten sich von den entsprechenden Aminosäuren ab. Die folgenden Aminosäurereste sind bevorzugt:

> Gly, Ala, Ser, Thr, Val, β-Ala, γ-Aminobutyrat, Asp, Glu, Asn, Aln, Nε-Acetyllysin, $N_\delta$-Acetylomithin, $N_\gamma$-Acetyldiaminobutyrat, $N_\alpha$-Acetyldiaminobutyrat.

Die Kurzschreibweise der Aminosäuren erfolgte nach der allgemein üblichen Schreibweise. Die Di- oder Tripeptidreste sind in ihrer chemischen Natur nach Säureamide und zerfallen bei der Hydrolyse in 2 oder 3 Aminosäuren. Die Aminosäuren in dem Di- oder Tripeptidrest sind durch Amidbindungen miteinander verbunden.

Bezüglich der Herstellung der Di- und Tripeptidreste wird ausdrücklich auf die EP 0 671 161 A1 der Firma Marbert verwiesen. Auch Beispiele für Di- und Tripeptidreste sind der Offenbarung der EP 0 671 161 A1 zu entnehmen.

Beispiele für $C_1$ - $C_4$-Alkylgruppen in den erfindungsgemäßen Verbindungen sind Methyl, Ethyl, n-Propyl, Isopropyl, n-Butyl, Isobutyl und tert.-Butyl. Bevorzugte Alkylgruppen sind Methyl und Ethyl, Methyl ist eine besonders bevorzugte Alkylgruppe. Bevorzugte $C_2$ - $C_4$-Hydroxyalkylgruppen sind die Gruppen 2-Hydroxyethyl, 3-Hydroxypropyl oder 4-Hydroxybutyl; 2-Hydroxyethyl ist eine besonders bevorzugte Hydroxyalkylgruppe.

Die erfindungsgemäßen farbverändernden Mittel enthalten diese Wirkstoffe bevorzugt in Mengen von 0,001 bis 2, insbesondere von 0,01 bis 0,5 Gew.-%, jeweils bezogen auf die gesamte Anwendungszubereitung.

Im Rahmen einer weiteren bevorzugten Ausführungsform enthalten die erfindungsgemäßen farbverändernden Mittel als Pflegestoff mindestens ein Mono- bzw. Oligosaccharid.

Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Da die eingesetzten Mono- bzw. Oligosaccharide üblicherweise aus natürlichen Rohstoffen wie Stärke gewonnen werden, weisen sie in der Regel die diesen Rohstoffen entsprechenden Konfigurationen auf (z.B. D-Glucose, D-Fructose und D-Galactose).

Weiterhin hat es sich als vorteilhaft erwiesen, wenn die erfindungsgemäßen Zubereitungen eine erhitzte, das heißt karamellisierte Zuckerart, vorzugsweise karamellisierten Rohrzucker enthalten.

Die Mono- bzw. Oligosaccharide sind in den erfindungsgemäßen Haarbehandlungsmitteln bevorzugt in einer Menge von 0,1 bis 8 Gew.-%, insbesondere 1 bis 5 Gew.-%, bezogen auf die gesamte Anwendungszubereitung, enthalten.

Im Rahmen einer elften bevorzugten Ausführungsform enthält die erfindungsgemäße Zubereitung (B) als Pflegestoff mindestens ein Silikonöl und/oder ein Silikongum.

Erfindungsgemäß geeignete Silikone oder Silikongums sind insbesondere Dialkyl- und Alkylarylsiloxane, wie

beispielsweise Dimethylpolysiloxan und Methylphenyl-polysiloxan, sowie deren alkoxylierte, quaternierte oder auch anionische Derivate.

Gemäß einer bevorzugten Ausführungsform enthalten die erfindungsgemäßen Zubereitungen eine Kombination aus einem flüchtigen und einem nichtflüchtigen Silikon. Flüchtig im Sinne der Erfindung sind solche Silikone, die ein Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning®1401, Dow Corning®1403 und Dow Corning®1501, jeweils Mischungen aus einem Cyclomethicone und einem Dimethiconol) erhältlich.

Gemäß einer besonders bevorzugten Ausführungsform wird als Komponente (A) ein Dialkylpolysiloxan oder eines seiner Derivate eingesetzt. Bevorzugt sind die Alkylgruppen Methyl, Ethyl, i-Propyl und n-Propyl. Dimethylpolysiloxan oder eines seiner Derivate wird besonders bevorzugt eingesetzt. Bevorzugte sind die Derivate des Dimethylplysiloxans, die aminofunktionell sind. Ein ganz besonders bevorzugtes Derivat ist unter der INCI-Bezeichnung Amodimethicone im Handel erhältlich.

Die erfindungsgemäßen Zubereitungen enthalten die Silikone bevorzugt in Mengen von 0,01 - 10 Gew.-%, insbesondere 0,1 - 5 Gew.%, bezogen auf die gesamte Anwendungszubereitung.

Im Rahmen einer weiteren Ausführungsform enthalten die erfindungsgemäßen Zubereitungen mindestens ein Lipid als Pflegestoff.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben.

Die erfindungsgemäßen Zubereitungen enthalten die Lipide bevorzugt in Mengen von 0,01 - 10 Gew.-%, insbesondere 0,1 - 5 Gew.%, bezogen auf die gesamte Anwendungszubereitung.

Im Rahmen einer weiteren Ausführungsform enthalten die erfindungsgemäßen Zubereitungen mindestens ein Ölkörper als Pflegestoff.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Din-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Diisotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I),

$$\begin{array}{l} CH_2O(CH_2CH_2O)_mR^1 \\ | \\ CHO(CH_2CH_2O)_nR^2 \\ | \\ CH_2O(CH_2CH_2O)_qR^3 \end{array} \qquad (D4\text{-}I)$$

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen farbverändernden Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Im Rahmen einer weiteren Ausführungsform enthalten die erfindungsgemäßen Zubereitungenein Enzym als Pflegestoff. Erfindungsgemäß besonders bevorzugte Enzyme sind ausgewählt aus einer Gruppe, die gebildet wird aus Proteasen, Lipasen, Transglutaminase, Oxidasen und Peroxidasen.

Obwohl jeder der in den verschiedenen Ausführungsformen genannten Pflegestoffe für sich alleine bereits ein zufriedenstellendes Resultat ergibt, sind im Rahmen der vorliegenden Erfindung auch alle Ausführungsformen umfasst, in denen die farbverändernden Mittel mehrere Pflegestoffe auch aus verschiedenen Gruppen enthalten.

Besonders bevorzugte Pflegestoffe im Sinne der vorliegenden Erfindung sind insbesondere Vitamin C, Ubiquinone, Panthenol und seine Derivate, Ectoin, Kamillenextrakt, Bisabolol, Olivenöl, Royal Gelee, Mung Bean Extrakt und Moringa Olifeira-Extrakt.

Weiterhin hat es sich als vorteilhaft erwiesen, wenn die erfindungsgemäßen Zubereitungen weiterhin ein natürlich vorkommendes Verdickungsmittel enthalten.

Bevorzugte Verdickungsmittel dieser Ausführungsform sind beispielsweise nichtionischen Guargums. Erfindungsgemäß können sowohl modifizierte als auch unmodifizierte Guargums zum Einsatz kommen. Nichtmodifizierte Guargums werden beispielsweise unter der Handelsbezeichnung Jaguar® C von der Firma Rhone Poulenc vertrieben. Erfindungsgemäß bevorzugte modifizierte Guargums enthalten $C_1$- bis $C_6$-Hydroxyalkylgruppen. Bevorzugt sind die Gruppen Hydroxymethyl, Hydroxyethyl, Hydroxypropyl und Hydroxybutyl. Derart modifizierte Guargums sind im Stand der Technik bekannt und können beispielsweise durch Reaktion der Guargums mit Alkylenoxiden hergestellt werden. Der Grad der Hydroxyalkylierung, der der Anzahl der verbrauchten Alkylenoxidmoleküle im Verhältnis zur Zahl der freien Hydroxygruppen der Guargums entspricht, liegt bevorzugt zwischen 0,4 und 1,2. Derart modifizierte Guar Gums sind unter den Handelsbezeichnungen Jaguar® HP8, Jaguar® HP60, Jaguar® HP120, Jaguar® DC 293 und Jaguar® HP105 der Firma Rhone Poulenc im Handel erhältlich.

Gemäß dieser Ausführungsform bevorzugt sind weiterhin Biosaccharidgums mikrobiellen Ursprungs, wie die Skleroglucangums oder Xanthangums, Gums aus pflanzlichen Exsudaten, wie beispielsweise Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Bevorzugte Hydroxyalkylcellulosen sind insbesondere die Hydroxyethylcellulosen, die unter den Bezeichnungen Cellosize® der Firma Amerchol und Natrosol® der Firma Hercules vertrieben werden. Geeignete Carboxyalkylcellulosen sind insbesondere die Carboxymethylcellulosen, wie sie unter den Bezeichnungen Blanose® von der Firma Aqualon, Aquasorb® und Ambergum® von der Firma Hercules und Cellgon® von der Firma Montello vertrieben werden.

Bevorzugt sind insbesondere Stärke und deren Derivate. Stärke ist ein Speicherstoff von Pflanzen, der vor allem in Knollen und Wurzeln, in Getreide-Samen und in Früchten vorkommt und aus einer Vielzahl von Pflanzen in hoher Ausbeute gewonnen werden kann. Das Polysaccharid, das in kaltem Wasser unlöslich ist und in siedendem Wasser eine kolloidale Lösung bildet, kann beispielsweise aus Kartoffeln, Maniok, Bataten, Maranta, Mais, Getreide, Reis, Hülsenfrüchte wie beispielsweise Erbsen und Bohnen, Bananen oder dem Mark bestimmter Palmensorten (beispielsweise der Sagopalme) gewonnen werden. Erfindungsgemäß einsetzbar sind natürliche, aus Pflanzen gewonnene Stärken und/oder chemisch oder physikalisch modifizierte Stärken. Eine Modifizierung läßt sich beispielsweise durch

Einführung unterschiedlicher funktioneller Gruppenan einer oder mehreren der Hydroxylgruppen der Stärke erreichen. Üblicherweise handelt es sich um Ester, Ether oder Amide der Stärke mit gegebenenfalls substituierten $C_1$- bis $C_{40}$-Resten. Besonders vorteilhaft ist eine mit einer 2-Hydroxypropylgruppe veretherte Maisstärke, wie sie beispielsweise von der Firma National Starch unter der Handelsbezeichnung Amaze® vertrieben wird.

Biosaccharidgums mikorbiellen Ursprungs, wie die Skleroglucangums oder Xanthangums, sind erfindungsgemäß ganz besonders bevorzugt.

Die erfindungsgemäßen Zubereitungen können neben den erfindungswesentlichen Komponenten weiterhin alle für solche Zubereitungen bekannten Wirk-, Zusatz- und Hilfsstoffe enthalten.

In vielen Fällen enthalten die Zubereitungen mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Hinsichtlich der kationischen Tenside sei an dieser Stelle auf die obigen Ausführungen verwiesen.

Ferner können die erfindungsgemäßen Zubereitungen weitere Wirk-, Hilfs- und Zusatzstoffe, wie beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- quatemierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wassserstoffperoxid und andere Oxidationsmittel,
- Treibmittel wie Propan-Butan-Gemische, $N_2O$, Dimethylether, $CO_2$ und Luft,
- Antioxidantien,

enthalten.

Die Zubereitungen enthalten die erfindungswesentlichen Komponenten erfindungsgemäß bevorzugt in einem

geeigneten wässrigen, alkoholischen oder wässrig-alkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind.

Unter wässrig-alkoholischen Lösungen sind im Sinne der vorliegenden Erfindung wässrige Lösungen enthaltend 3 bis 70 Gew.-% eines $C_1$-$C_4$-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Weiterhin können die erfindungsgemäßen Zubereitungen ein Reduktionsmittel enthalten. Beispiele für erfindungsgemäß bevorzugte Reduktionsmittel sind Natriumsulfit, Ascorbinsäure, Thioglykolsäure und deren Derivate, Natriumthionit, Alkalimetallcitratsalze und N-Acetyl-L-Cystein Ganz besonders bevorzugte Reduktionsmittel sind Alkalimetallcitratsalze, insbesondere Natriumcitrat, und N-Acetyl-L-Cystein. N-Acetyl-L-Cystein ist ein ganz besonders bevorzugtes Reduktionsmittel. Die Reduktionsmittel sind erfindungsgemäß vorzugsweise in der Zubereitung (A) enthalten.

Weiterhin können die erfindungsgemäßen Zubereitungen Alkalisierungsmittel, üblicherweise Alkali- oder Erdalkalihydroxide, Ammoniak oder organische Amine, enthalten. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Häufig werden in Färbemitteln Perlglanzpigmente eingesetzt. Erfindungsgemäß bevorzugte Perlglanzpigmente sind natürliche Perlglanzpigmente wie z.B. Fischsilber (Guanin/Hypoxanthin-Mischkristalle aus Fischschuppen) oder Perlmutt (aus vermahlenen Muschelschalen), monokristalline Perlglanzpigmente wie z.B. Bismutoxychlorid, sowie Perlglanzpigmente auf Basis von Glimmer oder Glimmer/Metalloxid. Durch den Einsatz der Perlglanzpigmente werden Glanz und gegebenenfalls zusätzlich Farbeffekte in den erfindungsgemäßen Mitteln erzielt. Die Farbgebung durch die in den Mitteln verwendeten Perlglanzpigmente beeinflusst das Farbergebnis der Färbung der Keratinfasern jedoch nicht.

Perlglanzpigmente auf Glimmer-Basis und auf Glimmer/Metalloxid-Basis sind erfindungsgemäß ebenfalls bevorzugt. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet. Geeignete Metalloxide sind u.a. $TiO_2$, $Cr_2O_3$ und $Fe_2O_3$. Durch entsprechende Beschichtung werden Interferenzpigmente sowie Farbglanzpigmente als erfindungsgemäße Perlglanzpigmente erhalten. Diese Perlglanzpigmentarten weisen neben einem glitzernden optischen Effekt zusätzlich Farbeffekte auf. Desweiteren können die erfindungsgemäß verwendbaren Perlglanzpigmente weiterhin ein Farbpigment enthalten, welches sich nicht von einem Metalloxid ableitet.

Die Korngröße der bevorzugt verwendeten Perlglanzpigmente liegt bevorzugt zwischen 1.0 und 100 μm, besonders bevorzugt zwischen 5.0 und 60.0 μm.

Besonders bevorzugte Perlglanzpigmente sind Pigmente, die von der Firma Merck unter den Handelsnamen Colorona® vermarktet werden, wobei die Pigmente Colorona® red-brown (47-57 Gew.% Muscovit Mica ($KH_2(AlSiO_4)_3$), 43-50 Gew.% $Fe_2O_3$ (INCI: Iron Oxides Cl 77491), <3 Gew.% $TiO_2$ (INCI: Titanium Dioxide Cl 77891), Colorona® Blackstar Blue (39-47 Gew.% Muscovit Mica ($KH_2(AlSiO_4)_3$), 53-61 Gew.% $Fe_3O_4$ (INCI: Iron Oxides Cl 77499)), Colorona® Siena Fine (35-45 Gew.% Muscovit Mica ($KH_2(AlSiO_4)_3$), 55-65 Gew.% $Fe_2O_3$ (INCI: Iron Oxides Cl 77491)), Colorona® Aborigine Amber (50-62 Gew.% Muscovit Mica ($KH_2(AlSiO_4)_3$), 36-44 Gew.% $Fe_3O_4$ (INCI: Iron Oxides Cl 77499), 2-6 Gew.% $TiO_2$ (INCI: Titanium Dioxide Cl 77891)), Colorona® Patagonian Purple (42-54 Gew.% Muscovit Mica ($KH_2(AlSiO_4)_3$), 26-32 Gew.% $Fe_2O_3$ (INCI: Iron Oxides Cl 77491), 18-22 Gew.% $TiO_2$ (INCI: Titanium Dioxide Cl 77891), 2-4 Gew.% Preussisch Blau (INCI: Ferric Ferrocyanide Cl 77510)), Colorona® Chameleon (40-50 Gew.% Muscovit Mica ($KH_2(AlSiO_4)_3$), 50-60 Gew.% $Fe_2O_3$ (INCI: Iron Oxides Cl 77491)) und Silk® Mica (>98 Gew.% Muscovit Mica ($KH_2(AlSiO_4)_3$)).

Die im bisherigen Verlauf der Anmeldung erwähnten weiteren Pflege-, Hilfs- oder Zusatzstoffe können dabei sowohl in der Zubereitung (A) als auch der Zubereitung (B) des erfindungsgemäßen Kits eingearbeitet werden. Auch einem Einsatz eines derartigen Wirkstoffs in beiden Zubereitungen kann erfindungsgemäß bevorzugt sein.

In der Regel genügt die Kraft des erfindungswesentlichen Wasserstoffperoxids zur Oxidierung der Farbstoffvorprodukte. Die Anwendungszubereitung kann aber auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Das eigentliche oxidative Färbemittel wird zweckmäßigerweise unmittelbar vor der Anwendung durch Mischung der Zubereitung (B) mit der Zubereitung (A), enthaltend die Farbstoffvorprodukte, hergestellt. Das dabei entstehende gebrauchsfertige Haarfärbepräparat sollte bevorzugt einen pH-Wert im Bereich von 5 bis 14, insbesondere von 7 bis 12, aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5

bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung (A) mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein weiterer Gegenstand der vorliegenden Erfindung ist Verfahren zur Farbveränderung keratinischer Fasern, bei dem ein Mittel, enthaltend mindestens eine Entwicklerkomponente und mindestens eine Kupplerkomponente, mit einer wässrigen Wasserstoffperoxidzubereitung, enthaltend mindestens eine Esterverbindung, die als Alkohol-Komponente einen gesättigten oder ungesättigten, verzweigten oder unverzweigten Wachsalkohol enthält, die Anwendungszubereitung auf die Fasern aufgetragen wird und nach einer Einwirkzeit wieder abgespült wird.

Dabei hat es sich als besonders vorteilhaft erwiesen, wenn die Einwirkzeit von 1 bis 60 Minuten, vorzugsweise 1 bis 15 Minuten, inbesondere 1 bis 5 Minuten, beträgt.

Die nachfolgenden Beispiele sollen den Gegenstand der vorliegenden Anmeldung verdeutlichen, ohne ihn in irgendeiner Weise zu beschränken.

Beispiele

Die Mengenangaben verstehen sich, soweit nichts anderes vermerkt ist, jeweils in Gewichtsprozent.

*1 Herstellung der Zubereitungen*

1.1 Herstellung der Farbrezepturen

| Rohstoff | Farbrezeptur A | Farbrezeptur B | Farbrezeptur C | Farbrezeptur D |
|---|---|---|---|---|
| Xanthan® FN | 0,1 | 0,1 | 0,1 | 0,1 |
| Ectoin | 0,1 | 0,1 | 0,1 | 0,1 |
| Ronacare®Tiliroside | 0,01 | 0,01 | 0,01 | 0,01 |
| Cetiol® V | 2,3 | 2,3 | 2,3 | 2,3 |
| Lanette® N | 14,0 | 14,0 | 14,0 | 14,0 |
| Cetearyl Alcohol | 3,9 | 3,9 | 3,9 | 3,9 |
| Cutina® GMS SE | 6,0 | 6,0 | 6,0 | 6,0 |
| Kokosamidopropylbetain 40 % | 2,0 | 2,0 | 2,0 | 2,0 |
| Monoethanolamin | 0,2 | 0,4 | 0,3 | 0,3 |
| Karamelzuckersirup 75 % | 0,1 | 0,1 | 0,1 | 0,1 |
| Ascorbinsäure I | 0,1 | 0,1 | 0,1 | 0,1 |
| Natriumsulfit | -- | 0,3 | -- | 0,2 |
| Monoethanolamin | 3,0 | 3,0 | 3,0 | 3,0 |
| p-Toluylendiaminsulfat | 0,7 | 1,5 | 1,0 | 1,0 |
| m-Aminophenol | 0,1 | 0,2 | 0,1 | 0,04 |
| 2-Amino-4-Hydroxyethylaminoanisolsulfat | 0,04 | 0,02 | -- | -- |
| Resorcin | 0,3 | 0,6 | 0,3 | 0,1 |

(fortgesetzt)

| Rohstoff | Farbrezeptur A | Farbrezeptur B | Farbrezeptur C | Farbrezeptur D |
|---|---|---|---|---|
| 2-Methylresorcin | -- | -- | 0,1 | 0,3 |
| 2-Amino-3-hydroxypyridin | -- | -- | -- | 0,1 |
| Wasser | ad 100 | ad 100 | ad 100 | ad 100 |

1.2 Herstellung der Oxidationsmittelzubereitungen

| Rohstoff | Variante I (Vergleich) | Variante II (Erfindung) | Variante III (Vergleich) |
|---|---|---|---|
| Natriumbenzoat | 0,04 | 0,04 | 0,04 |
| Dinatriumpyrophosphat | 0,1 | 0,1 | 0,1 |
| Turpinal® SL | 0,3 | 0,3 | 0,3 |
| Kaliumhydroxid 50 % | 0,1 | 0,1 | 0,1 |
| Isopropylmyristat | 0,8 | 0,8 | 0,8 |
| Cetearyl Alcohol | 4,0 | 4,0 | 4,0 |
| Eumulgin® B 2 | 1,0 | 1,0 | 1,0 |
| Paraffinum Liquidum | 0,5 | -- | -- |
| Bienenwachs | -- | 0,5 | -- |
| Meadowfoam Seed Oil | -- | -- | 0,5 |
| Wasserstoffperoxid (50%ig) | 5,2 | 5,2 | 5,2 |
| Wasser | ad 100 | ad 100 | ad 100 |

1.3 Eingesetzte Rohstoffe

| | |
|---|---|
| Cetiol® V | Ölsäuredecylester (INCI-Bezeichnung: Decyl Oleate) (Cognis) |
| Cutina® GMS-SE | INCI-Bezeichnung: Glyceryl Stearate SE (Cognis) |
| Eumulgin® B2 | Cetylstearylalkohol mit ca. 20 EO-Einheiten (INCI-Bezeichnung: Ceteareth-20) (Cognis) |
| Lanette® N | Gemisch aus 90 Gewichtsteilen Cetylstearylalkohol und 10 Gewichtsteilen Natriumcetylstearylsulfatl (INCI-Bezeichnung: Cetearyl Alcohol, Sodium Cetearyl Sulfate) (Cognis) |
| Ronacare®Tiliroside | Mischung von Tiliroside und Sorbitol (ca. 4-5Gew% Tiliroside; INCI-Bezeichnung: Sorbitol, Tiliroside) (Merck) |
| Turpinal® SL | 1-Hydroxyethan-1,1-diphosphonsäure (ca. 58 - 61% Aktivsubstanzgehalt; INCI-Bezeichnung: Etidronic Acid, Aqua (Water)) (Solutia) |
| Xanthan® FN | INCI-Bezeichnung: Xanthan Gum (Jungbunzlauer) |

*2 Ausfärbungen*

50g der Farbrezepturen A bis C wurden jeweils mit 100g der Oxidationsmittelzubereitungen der Varianten I, II und III gemischt. Die resultierenden Anwendungszubereitungen wurden jeweils auf eine Strähne Büffelbauchhaar aufgetragen und dort für 30 Minuten bei Raumtemperatur belassen.

Anschließend wurden die Haare gründlich mit einem handelsüblichen Shampoo gespült.

*3 Auswertung*

Vor und nach dieser Behandlung wurden die Fasern farbmetrisch vermessen und Ergebnisse der Messungen mithilfe des CIELAB-Farbenraums quantifiziert.

Der L-Wert steht dabei für die Helligkeit der Färbung (schwarz-weiß-Achse); je größer der Wert für L ist, desto heller ist die Färbung.

Farbunterschiede werden mittels des ΔE-Wertes charakterisiert, der entsprechend Gleichung (I) berechnet wird:

$$\Delta E = \sqrt{(\Delta L)^2 + (\Delta a)^2 + (\Delta b)^2} \quad (I)$$

Bei den folgenden Werten wurden jeweils die Messdaten, die für die Variante I (Paraffinöl) erhalten wurden als Standard verwendet.

3.1 Bestimmung der ΔL-Werte

| | Variante II Bienenwachs (Erfindung) | Variante III Meadowfoam Seed Oil |
|---|---|---|
| Rezeptur A | -3,6 | -0,3 |
| Rezeptur B | -3,9 | 0,2 |
| Rezeptur C | -3,3 | -0,3 |

Für die Variante III (Meadowfoam Seed Oil) wurden Werte ermittelt, die denen der Variante I (Paraffinöl) relativ nahe kommen (Differenz nahe bei null). Dementsprechend besteht nur ein geringer Unterschied in der Farbintensität der Ausfärbungen der Varianten I und III.

Im Gegensatz dazu zeigen die deutlich negativen ΔL-Werte, die sich bei den Berechnungen für die Rezepturen mit Bienenwachs (Variante II) ergaben, an, dass die resultierenden Färbungen wesentlich dunkler ausgefallen sind, als die der Referenz (Paraffinöl).

3.2 Bestimmung der ΔE-Werte

| | Variante II Bienenwachs (Erfindung) | Variante III Meadowfoam Seed Oil |
|---|---|---|
| Rezeptur A | 4,0 | 0,8 |
| Rezeptur B | 3,0 | 0,5 |
| Rezeptur C | 3,4 | 0,9 |

Auch an den ΔE-Werten lässt sich ablesen, dass die Färbungen der Varianten I und III relativ vergleichbar sind, während die Färbung der Variante II sich deutlich von diesen abhebt

## Patentansprüche

**1.** Kit zur Färbung keratinischer Fasern, bestehend aus

- einer ersten Zubereitung (A), enthaltend mindestens eine Entwicklerkomponente und mindestens eine Kupplerkomponente, und
- einer zweiten Zubereitung (B) in Form einer wässrigen Wasserstoffperoxidzubereitung, umfassend mindestens

eine Esterverbindung, die als Alkohol-Komponente einen gesättigten oder ungesättigten, verzweigten oder unverzweigten Wachsalkohol enthält.

2. Kit nach Anspruch 1, **dadurch gekennzeichnet, dass** der Fettalkohol Triacontan-1-ol ist.

3. Kit nach einem der Ansprüche 1 oder 2, **dadurch gekennzeichnet, dass** die Esterverbindung als Säurekomponente Palmitin oder Cerotinsäure enthält.

4. Kit nach einem der Ansprüche 1 bis 3, **dadurch gekennzeichnet, dass** die Esterverbindung aus Bienenwachs gewonnen wird.

5. Kit nach einem der Ansprüche 1 bis 3, **dadurch gekennzeichnet, dass** Zubereitung (B) Bienenwachs enthält.

6. Kit nach einem der Ansprüche 1 bis 5, **dadurch gekennzeichnet, dass** die erste Zubereitung (A) weiterhin mindestens ein Flavonoid der Formel (I)

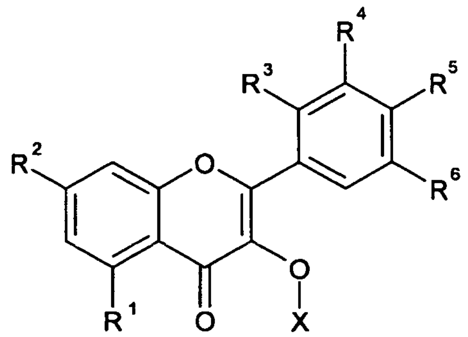

(I)

wobei $R^1$ bis $R^6$ stehen unabhängig voneinander für ein Wasserstoffatom, eine Hydroxygruppe, ein Hydroxy-($C_1$-bis $C_4$)-alkoxygruppe oder eine Sulfatgruppe und X steht für einen gegebenenfalls substituierten Glucosidrest, enthält.

7. Kit nach einem der Ansprüche 1 bis 6, **dadurch gekennzeichnet, dass** eine der Zubereitungen ein natürlich vorkommendes Verdickungsmittel, vorzugsweise Xanthangum, enthält.

8. Kit nach einem der Ansprüche 1 bis 7, **dadurch gekennzeichnet, dass** eine der Zubereitungen weiterhin mindestens ein Mono- und/oder ein Oligosaccharid enthalten.

9. Verfahren zur Farbveränderung keratinischer Fasern, **dadurch gekennzeichnet, dass**

- ein Mittel, enthaltend mindestens eine Entwicklerkomponente und mindestens eine Kupplerkomponente, mit einer wässrigen Wasserstoffperoxidzubereitung, umfassend mindestens eine Esterverbindung, die als Alkohol-Komponente einen gesättigten oder ungesättigten, verzweigten oder unverzweigten Wachsalkohol enthält, vermischt wird,
- die Anwendungszubereitung auf die Fasern aufgetragen wird und
- nach einer Einwirkzeit wieder abgespült wird.

## Claims

1. Kit for dyeing keratinic fibers, comprising

- a first preparation (A) which contains at least one developer component and at least one coupler component, and
- a second preparation (B) in the form of an aqueous hydrogen peroxide preparation, including at least one ester compound which contains a saturated or unsaturated, branched or unbranched wax alcohol as the alcohol component.

2. Kit according to Claim 1, **characterized in that** the fatty alcohol is triacontan-1-ol.

3. Kit according to one of Claims 1 or 2, **characterized in that** the ester compound contains palmitic acid or cerotinic acid as the acid component.

4. Kit according to one of Claims 1 to 3, **characterized in that** the ester compound is obtained from beeswax.

5. Kit according to one of Claims 1 to 3, **characterized in that** preparation (B) contains beeswax.

6. Kit according to one of Claims 1 to 5, **characterized in that** the first preparation (A) also contains at least one flavonoid of formula (I)

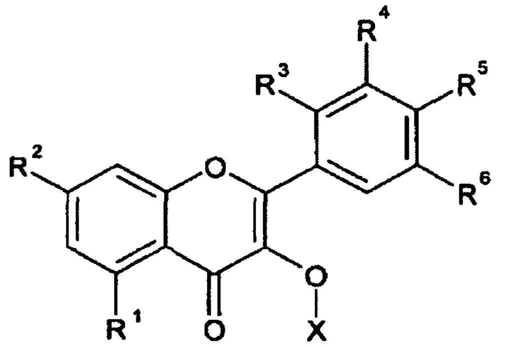

(I),

where $R^1$ to $R^6$ independently stand for a hydrogen atom, a hydroxy group, a hydroxy-($C_1$ to $C_4$) alkoxy group, or a sulfate group, and X stands for an optionally substituted glucoside radical.

7. Kit according to one of Claims 1 to 6, **characterized in that** one of the preparations contains a naturally occurring thickening agent, preferably xanthan gum.

8. Kit according to one of Claims 1 to 7, **characterized in that** one of the preparations also contains at least one monosaccharide and/or one oligosaccharide.

9. Method for changing the color of keratinic fibers, **characterized in that**

- an agent containing at least one developer component and at least one coupler component is mixed with an aqueous hydrogen peroxide preparation which includes at least one ester compound which contains a saturated or unsaturated, branched or unbranched wax alcohol as the alcohol component,
- the application preparation is applied to the fibers, and
- the application preparation is rinsed off after an exposure period.

## Revendications

1. Kit destiné à la coloration de fibres de kératine, consistant en

- une première composition (A) contenant au moins un composant développeur et au moins un composant coupleur, et
- une deuxième composition (B) se présentant sous forme d'une composition de peroxyde d'hydrogène aqueuse comprenant au moins un composé ester qui contient comme composant alcool un alcool de cire saturé ou non saturé, ramifié ou non ramifié.

2. Kit selon la revendication 1, **caractérisé en ce que** l'alcool gras est du triacontan-1-ol.

3. Kit selon l'une des revendications 1 ou 2, **caractérisé en ce que** le composé ester contient comme composant acide de l'acide palmitique ou cérotique.

4. Kit selon l'une des revendications 1 à 3, **caractérisé en ce que** le composé ester est obtenu à partir de cire d'abeille.

**5.** Kit selon l'une des revendications 1 à 3, **caractérisé en ce que** la composition (B) contient de la cire d'abeille.

**6.** Kit selon l'une des revendications 1 à 5, **caractérisé en ce que** la première composition (A) contient en outre au moins un flavonoïde de la formule (I)

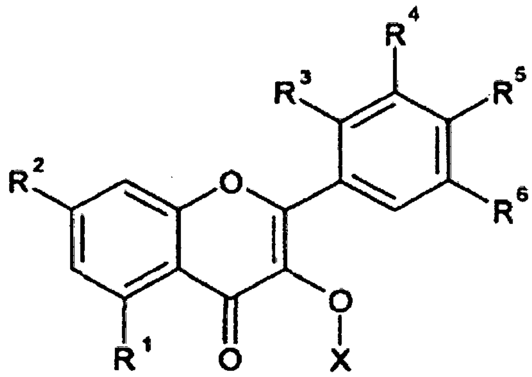

(I)

dans laquelle $R^1$ à $R^6$ sont indépendamment un atome d'hydrogène, un groupe hydroxy, un groupe hydroxy($C_1$ à $C_4$)alcoxy ou un groupe sulfate et X représente un radical glucoside éventuellement substitué.

**7.** Kit selon l'une des revendications 1 à 6, **caractérisé en ce que** l'une des compositions contient un épaississant naturel, de préférence de la gomme de xanthane.

**8.** Kit selon l'une des revendications 1 à 7, **caractérisé en ce que** l'une des compositions contient en outre au moins un monosaccharide et/ou un oligosaccharide.

**9.** Procédé de changement de la couleur de fibres de kératine, **caractérisé en ce que**

- une composition, comprenant au moins un composant développeur et au moins un composant coupleur, est mélangé à une composition de peroxyde d'hydrogène aqueuse comprenant au moins un composé ester qui contient comme composant alcool un alcool de cire, saturé ou non saturé, ramifié ou non ramifié,
- la composition d'application est appliquée sur les fibres et,
- après avoir laissé agir, est à nouveau éliminée par rinçage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 19723538 C1
- US 20040181883 A1
- US 20070033744 A1
- US 4844886 A
- US 20060121070 A1
- US 20050039270 A1
- EP 998908 A2
- WO 0269926 A
- WO 06099930 A
- EP 0671161 A1
- DE OS19756454 A